# EUROPEAN PATENT SPECIFICATION

(11) **EP 2 208 482 B1**
(45) Date of publication and mention of the grant of the patent: **30.09.2015**
(21) Application number: 08839047.1
(22) Date of filing: 10.10.2008
(51) Int. Cl.: A61F 2/82, A61L 31/10, A61L 31/16

(54) **AN ARTIFICIAL STENT AND ITS PREPARATION METHOD**
KÜNSTLICHER STENT UND HERSTELLUNGSVERFAHREN DAFÜR
STENT ARTIFICIEL ET PROCÉDÉ DE PRÉPARATION DUDIT STENT

(30) Priority: 12.10.2007 CN 200710047025
(43) Date of publication of application: 21.07.2010
(73) Proprietor: Shanghai MicroPort Medical (Group) Co., Ltd., Shanghai 201203 (CN)
(72) Inventor: LUO, Qiyi, Shanghai 201203 (CN); TANG, Zhirong, Shanghai 201203 (CN); ZHENG, Buzhong, Shanghai 201203 (CN)
(74) Representative: Kling, Simone
(86) International application number: PCT/CN2008/001712
(87) International publication number: WO 2009/049494

(56) References cited:
- WO-A2-2005/123114
- WO-A2-2007/016524
- CN-A- 1 360 951
- CN-A- 1 372 023
- CN-A- 1 408 446
- US-A1- 2006 030 927
- US-A1- 2006 167 540
- US-A1- 2007 187 862

## Description

### FIELD OF INVENTION

The present invention relates to the field of biomaterials and medical instruments, in particular, to an artificial stent and its preparation method.

### BACKGROUND OF THE INVENTION

Silk is a natural protein comprising 18 species of amino acids including glycine, alanine, serine, etc. This protein is a natural fibrous high polymer substantially consisting of repeated polypeptide units of Gly-Ala-Gly-Ala-Ser. The structural properties of silk dominate its bioactivities. The major amino acids contained in silk have a particular effect on human bodies.

Silk is abundantly available in China. China is one of the biggest silk providers in the world, was the first to introduce silk into industry. For thousands of years, silk is developed and used as materials for clothes and finery. However, silk fibroin has been extensively studied for its application as a food additive or in cosmetics, since it was found that silk fibroin has unique physical and chemical properties as well as excellent compatibilities for human body. Surgery suture being made of silk has been applied for many years, indicating that silk can be used in other fields, and is safe and compatible to human and does not bring allergic reactions.

The advantageous physical and chemical properties of silk fibroin, especially the good bio-compatibility, confer the potential of silk as being a novel bio-material. Silk fibroin does not present a complicated synthesis techniques and provides a simple purification process, as compared to other existing biomedical materials. It was showed in various animal models and clinical trials that silk fibroin is non-toxic and non-irritative and well bio-compatible. Therefore, silk fibroin can be applied as a medical material in various fields. At present, silk fibroin is used in enzyme-immobilized electrodes, wound paste materials, anticoagulation materials, dialysis membrane, contact lenses, and the like, as well as in surgery sutures, food additives and cosmetics. Furthermore, the potential of silk fibroin to be used in cell culture medium, artificial skin, and materials for control-released drugs has also been contemplated.

Cardio-cerebrovascular diseases are the leading threat to elderly people, and lead to hundreds of thousands of deaths or disabilities annually. Since the 1970s, transluminal balloon angioplasty has been widely used to treat vascular stenosis caused by atherosis. Though the immediate efficacy of coronary angioplasty is satisfactory, the incidence of complications, especially re-stenosis, is very high, presenting a limitation on its wide application in clinic. In the procedure of intravascular stenting, a stent is implanted at the location of stenosis with a concurrent balloon angioplasty. The stent resists the re-stenosis caused by vascular delamination or elastic recoil, thus significantly decreases the complications of acute or subacute ischemia. However, long-term implantation of stents stimulates the immigration and proliferation of smooth muscle cells, resulting in intimal hyperplasia which inturn leads to re-stenosis.

Re-stenosis in stents is a rather complex process involving a number of interaction mechanisms, in which the intravascular thrombosis, the immigration and proliferation of vascular smooth muscle cells and the hyperplasia of extracellular matrix are the major causes of re-stenosis in stents. Thus, anti-thrombosis and anti-hyperplasia drugs can efficiently prevent and treat re-stenosis. Given the low efficiency and high side effects of systemic administration, local intravascular sustained-release dosing can be a preferred alternative. Recently, stents coated by rapamycin and paclitaxel have been successfully used in clinic, in particular to treat re-stenosis in stents, indicating that drug-coated stents have a great potential in the treatment of re-stenosis.

Though existing drug-coated stents, as compared to uncoated stents, have been greatly improved the treatment of vascular re-stenosis, results from long-term analysis showed that drug-coated stents did not increase the survival rate of patients and might result in some adverse effects, such as delayed thrombus that may be fatal, and chronic inflammatory reactions resulted from bio-inert polymers. There is a great need for scientists, physicians and manufacturers of medical instruments to find a solution that overcomes the disadvantages of drug-coated stents while maintaining the efficiency of preventing re-stenosis.

US2007/0187862 describes materials produced from a silk fibroin solution of 10 to 30 wt%. These materials, e.g. foams or films, can contain therapeutic agents and may be used in a variety of medical applications, e.g. drug delivery devices, controlled release systems and scaffolds for tissue regeneration. Films of silk fibroin may be casted and used to coat articles such as a stent.

### BRIEF DESCRIPTION OF THE INVENTION

The present invention is based on the following concept: silk fibroin is a natural bio-material with great bio-compatibility, especially blood-compatibility, and can be absorbed and metabolized slowly by human body without adverse side effects. The inventor applied silk fibroin as a coating material onto artificial stents. Such a stent is capable of carrying drugs, and achieves the targeting delivery and sustained release of drugs, while avoids certain adverse effects of conventional drug-coated stents, such as delayed thrombus which lead to deaths, and the chronic inflammatory reactions resulted from bio-inert polymers.

An object of the invention is to provide an artificial stent with a drug coating which can be conveniently loaded with various drugs. The drug coating is well bio-compatible, and can be absorbed and metabolized slowly by human body without adverse side effects. Thus the targeting sustained-release of drugs is achieved while cost of stents is reduced.

Another object of the invention is to provide a method of preparing the artificial stent as described above.

The body of the artificial stent used in this invention mainly includes vascular stents, comprising coronary artery stents, carotid artery stents, intracranial vascular stents, main artery stents, peripheral vascular stents, which are commercially available. The silk fibroin may be exacted and prepared from silk using conventional methods, and also is commercial available. Unless indicated otherwise, other materials and adjuvants are those routinely used in the art and commercial available.

The artificial stent of the present invention is comprised of a stent body and a coating on the surface of the stent body, wherein the coating comprises a drug-loaded layer including silk fibroin and a drug, as defined in claim 1.

The coating material is mainly silk fibroin, and comprises one or more other ingredients as needed, such as collagen, gelatin, chitosan, sodium alginate, hyaluronic acid.

The drug-loaded layer has a microporous structure substantially formed with silk fibroin, in which a drug is loaded. The microporous structure substantially consists of interconnected interspace and dense micropores, formed with silk fibroin. Thus, thus microporous structure can be easily loaded with various drugs.

More preferably, the coating further comprises a compact base-layer formed with silk fibroin, which is located between the stent body and the drug-loaded layer. The coating material can firmly attached to the stent body, with an addition of the dense base-layer. The integrity of the coating can thus be maintained during operation of the stent, without detachment or cracking, thus facilitating the targeted-delivery and the sustained-released of the drug.

Still more preferably, the coating further comprises an outer-layer to control the speed of drug release.

The drug loaded as described above can vary, for example water-soluble or lipid-soluble chemical drugs, preferably proteinous drugs and/or nucleic acid drugs, which inhibits the activation of platelets, prevents thrombosis, prevents the immigration or proliferation of smooth muscle cells, promotes the proliferation of endothelial cells, and the like.

The manufacture of the present artificial stent mainly involves the formation of the microporous coating of silk fibroin, which may utilize water-soluble polymer pore-forming, salting out, phase separation, or combinations of the above. The methods for the manufacture of the artificial stent of the invention are illustrated as below.

A method for the manufacture of the artificial stent of the invention comprises the following steps:
1) Silk fibroin is dissolved in water or an organic solvent and mixed evenly, resulting in a silk fibroin solution of 1%wt-20%wt. A aqueous gelatin solution is added and mixed evenly, centrifuged and the supernatant collected.
2) The surface of the stent body is evenly coated with the supernatant resulted in step 1). Then the stent is denatured with heat or chemical reagents, washed with purified water, dryed, placed into purified water, and boiled until gelatin in the coating dissolved out completely. Then the stent is washed with purified water. The microporous structure would be formed after drying the coat.
3) The stent obtained in step 2) is placed into the solution of a drug, so as to load the drug into the micropores in the coating. Finally, the stent is removed and allowed to dry.

In the method above, the concentration of gelatin solution in step 1) is preferably 5-20% wt, and the volumetric ratio of the gelatin solution to the solution of silk fibrioin is no more than 1:1.

In the method above, a step of γ-ray irradiation, vacuum drying-heat treatment, and treatment with a monohydroxy alcohol or a polyhydroxy alcohol or combinations thereof may be added after step 2), so as to allow the indissolubility of the silk fibroin coating resulted from the alteration of crystal structure.

In the method above, upon boiling in step 2), gelatin dissolves out completely from the coating, thereby interconnected interspaces and dense micropores in the coating are formed in the coating of stents.

Another method for the manufacture of the artificial stent of the invention comprises the following steps.
1) Silk fibroin is dissolved in water or an organic solvent and mixed evenly, resulting in a solution of silk fibroin of 1 %wt-20%wt.
2) The solution of silk fibroin obtained in step 1) is evenly coated on the surface of the stent body, and denatured with heat or chemical reagents. Then the stent is soaked with purified water, and then frozen and warming dried, so as to allow the formation of microporous structure in the coating;
3) The stent obtained in step 2) is placed into the solution of a drug, so as to load the drug into the micropores in the coating. Then the stent is removed and allowed to dry.

In the method above, the solution of silk fibroin can be uniformly coated on the surface of the stent body by dipping or spraying.

In the method above, the treatment of frozen-warming-drying in step 2) may be as follows. Firstly, the temperature is decreased to -40°C∼ -80°C according to a programmed scheme with a rate of -0.5°C/min ∼ -5°C/min, allowing the crystallization of water in the coating. Then the temperature was increased to room temperature according to a programmed scheme with a rate of -0.1°C/min ∼ -2°C/min while maintaining the high-vacuum, allowing complete drying of the coating. In the process of freezing and warming-drying, the porosity and the structure of the pores in the coating of the stent can be adjusted by adjusting the parameters in the freezing process.

In the method above, a step of γ-ray irradiation, vacuum drying-heat treatment, and treatment with a monohydroxy alcohol or a polyhydroxy alcohol or combinations thereof may be added after step 2), so as to allow the indissolubility of the silk fibroin coating resulted from the alteration of crystal structure.

In the method above, the drug loaded in step 3) is preferably a proteinous drug and/or a nucleic acid drug. The drug can be loaded in the micropores of coating by soaking-infiltration or electrophoresis.

A method for the formation of a dense base-layer on the artificial stent comprises the steps as below:
1) Silk fibroin is dissolved in water or an organic solvent and mixed evenly, resulting a solution of silk fibroin of 1%wt-20%wt.
2) The solution of silk fibroin as described above is mixed evenly with a drug, coated on the surface of the stent body by dipping or spraying, and allowed to dry.

In the methods as described above, the concentration of the solution of silk fibroin is 1-5% wt. Certain concentration of additives can be added into the solution of silk fibroin obtained in step 1) as required, such as one or more of collagen, gelatin, chitosan, sodium alginate, hyaluronic acid, and the like.

The invention provides a method for preparing a stent with a porous coating of silk fibroin. The method is less time-consuming and is performed under mild conditions. The micropores in the coating are used as the reservoir of proteinous or nucleic acid drugs. The drug loaded in the coating of silk fibroin is sustainably released, improving the availability of the drug at the location of target vessel. Silk fibroin in the coating of the stent is a natural protein with great bio-compatibility, and can be absorbed and metabolized slowly by human body without adverse side effects, vascular stenosis is thus prevented and treated with improved safety and efficacy. Furthermore, certain adverse effects of conventional drug-coated stents, such as delayed thrombus which maybe fatal and chronic inflammatory reactions resulted from bio-inert polymers, are avoided.

The following advantageous effects are achieved with the artificial stent of the invention: 1) silk fibroin in the coating of stents is a natural protein with great bio-compatibility. It can be absorbed and metabolized slowly by human body without adverse side effects; 2) silk fibroin is widely available with low price. The present method is performed under mild conditions, facilitating the industrialization; 3) the coating material made of silk fibroin has a high strength, thus the coating would not detach or crack during operation of the stent; 4) the microporous coating of the stent may be used as a carrier to load various drugs, to achieve a targeted sustained-release, decreasing the cost for the manufacture of the stent; 5) the release profile of the drug can be controlled as needed, by accurately designing the structure of the coating.

### BRIEF DESCRIPTION OF THE DRAWINGS

Figure 1 is the electron microscope photograph of the whole porous stent.
Figure 2 is the electron microscope photograph of the surface of the porous stent.
Figure 3 is the electron microscope photograph of the cross-section of the coating of the porous stent.

### DESCRIPTION OF THE PREFERRED EMBODIMENTS

### Example 1 (not according to the invention)

5g of silk was added into 200 ml of 2% aqueous Na₂CO₃ and was treated at 98°C for 60min. The process as described above was repeated three times. After drying, the resulted substance was dissolved in 50% solution of lithium bromide. The resulted solution as described above was filtrated with multi-layer nonwoven fabrics to remove contaminants. Then the solution was placed in a dialysis tubing, and dialyzed for three days with flowing water, resulting a 1.5% solution of silk fibroin. A stainless steel stent body was washed with acetone and then purified water, and was allowed to dry. Subsequently, the solution of silk fibroin was evenly sprayed on the stent body. The resulted coated-stent was placed in the vacuum drying oven at 37°C for 24h. The coated-stent was placed in ethylene glycol (analytical grade) for 72h and washed with distilled water. Then the coated-stent was placed again in the vacuum drying oven at 37°C for 24h. Thus a dense base-layer was formed with silk fibroin on the stent body.

### Example 2

100ml 20% solution of silk fibroin was prepared, and 100ml 10% aqueous gelatin solution was added, mixed evenly. Cryo-centrifugation was performed and the supernatant was collected. A stainless steel stent body was soaked with the mixed solution as described above, and then dried at 20°C for 20h. The process above was repeated until the weight of the coating reached 1000ug, resulting in a even coating formed on the surface of the stent body. The coated-stent was placed in 25% aqueous glycerol solution for 1h. Subsequently, the stent was washed with water, and placed in the vacuum oven for 12h drying at 60°C. The coated-stent was placed in purified water for boiling for 2 hours. The process above was repeated three times, until gelatin in the coating was dissolved out completely. Then, the stent was washed with water and dried, thereby obtaining a stent with a coating containing uniform pores. Stent with a coating having different porosities can be obtained by adjusting the ratio of silk and gelatin.

### Example 3

5% solution of silk fibroin was prepared, and then sprayed on the surface of the stent body, with a rate of 0.25ml/min and duration of 30s. The process above was repeated until the weight reached 200ug, and a continuous base layer was thus formed on the surface of the stent body after drying. The stent with the base layer was placed in 80% aqueous ethanol solution for 24 hours, washed with water and vacuum-dried at room temperature. Furthermore, a mixed solution of 5% silk fibroin and 2.5% chitosan was sprayed on the stent with the base layer, until the coating weight reached 1000ug, and then allowed to air-dry. Subsequently, the stent was soaked with water, and immediately placed in deep-freezer at -80°C for 2 hours. Then, it was immediately transferred in a pre-cooled lyophilizer and lyophilized for 18h, thereby obtaining a coating structure with micropores. Then the coated-stent with microporous layer was placed in an electric oven thermostat at 60°C for 24h, obtaining a stent with a water-insoluble microporous coating.

### Example 4

Several 5ml Eppendorf tubes were prepared and each added 4ml of anhydrous ethanol. These tubes were placed in a 500ml beaker. 300 ml of anhydrous ethanol was added into the beaker, submerging the tubes. The beaker was sealed by parafilm and placed in a deep-freezer at -80°C overnight until use. The stent body was ultrasonically washed with acetone and purified water separately for three times and then dried at 50°C. A 100ug silk fibroin base layer was coated on the stent body by spraying, and then treated with ethanol for 24h. Subsequently, silk fibroin solution was sprayed on the stent with the base layer, until the weight reached 800ug, and then air-dried. The stent rinsed with water for 30 min, and then immediately placed in the Eppendorf tube containing anhydrous ethanol at -80°C. The Eppendorf tube was placed in a cryogenic refrigerator for temporary stock. Subsequently, a lyophilizer was turned on and the pre-freeze temperature was set on -40°C. The Eppendorf tube was quickly transferred from cryogenic refrigerator to the cold trap of the lyophilizer once the set temperature was achieved. The main process of drying was immediately started by vacuuming. The drying of the stent ended after 24h, resulting in a stent with a white microporous coating. The stent was placed in a glass desiccator for preservation.

### Example 5

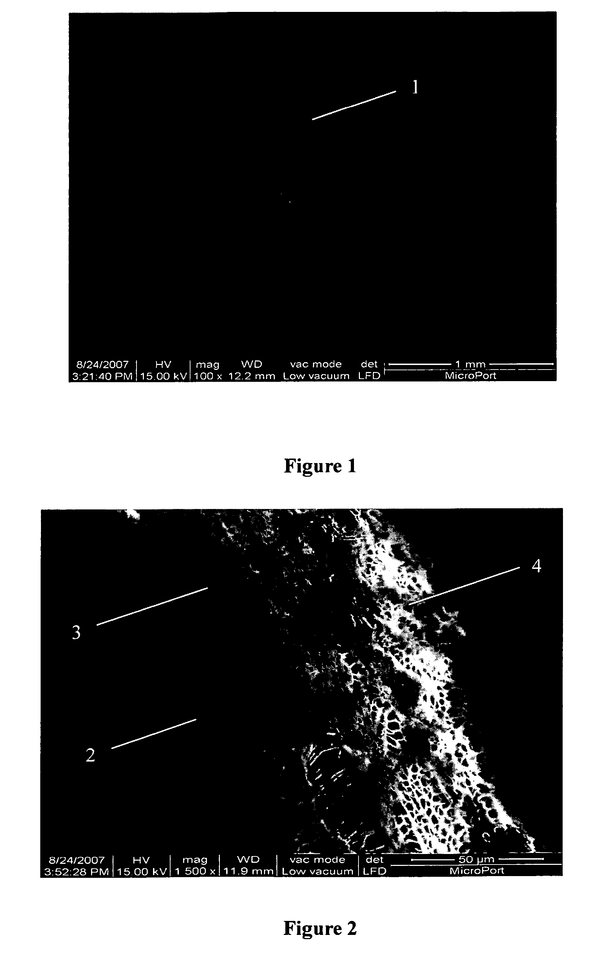

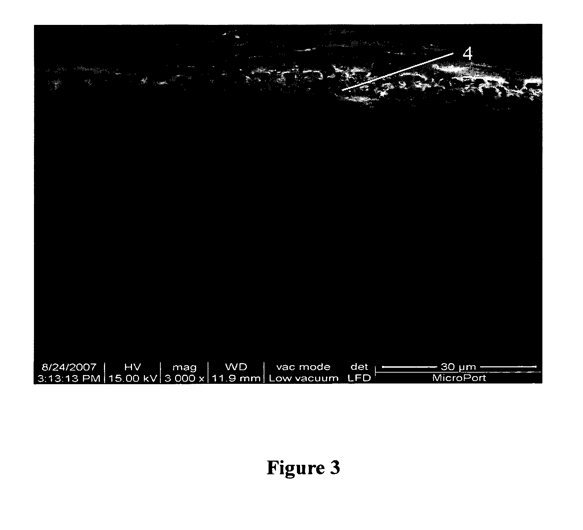

50ml of mixed solution containing 2% silk fibroin and 1% gelatin was prepared, and then sprayed on the surface of a stent body, with a rate of 0.2ml/min and duration of 80s, until the weight reached 1000ug. Subsequently, the stent was soaked with water for 1-5min. Then the stent was immediately transferred into a lyophilizer and rapidly cooled to -40°C. Lyophilization under high vacuum was performed for 20h, obtaining a stent with a microporous coating. The stent as described above was treated with ethanol for 10 hours, washed and then dried. Then, the coated-stent was placed in hot water for boiling for 2 hours, so that gelatin in the coating was dissolved out. Accordingly, a coating with micropores interconnected on the surface of the stent was formed. The stent with the microporous coating was submerged in the solution of a plasmid encoding VEGF protein for two-hours of leaching under reduced pressure. The stent was removed from the solution and washed with purified water to remove drugs attached on the surface. A vacuum-drying was performed for 12 hours, resulting in the artificial stent illustrated in Figure 1. Figure 2 is a local electron microscopy photograph at location 1 on the surface of the artificial stent in Figure 1. Fig. 2 shows the dense base layer 2 formed with silk fibroin, the interspace 3 interconnected in the coating, and the dense microspores 4 on the surface of the coating. Fig. 3 also shows the dense microspores 4 on the surface of the coating.

### Example 6

5g of silk was added into 100ml 0.6% NaOH solution, treated at 60°C for 1h and then washed with purified water for three times. Subsequently, it was dried and dissolved in 30% aqueous Mg(NO₃)₂ solution. The solution was filtrated and uniformly sprayed on the stent body, resulting in 1200 ug of coating. The coated-stent was placed in vacuum drying oven for 24-hour drying at 37°C. The stent was then placed in 60% aqueous ethanol solution for eight-hours of desalting. Subsequently, the coated-stent was placed in methanol solution (analytical grade) for 24h, washed with distilled water for three times and dried in vacuum drying oven for 24 hours, resulting in a stent with a white microporous coating. Stents containing micropores with a different porosities can be obtained by adjusting the content of silk fibroin in the aqueous Mg(NO₃)₂ solution. The stent containing micropores was fixed at the anode of low-voltage electrophoresis, which is inserted into a solution of adenovirus encoding FGF protein. The electrophoresis was performed at 5V for 35min. Then the stent was removed and washed with purified water to remove drugs attached on the surface. Vacuum drying was performed for 12h, resulting in a stent loaded with a nucleic acid drug.

## Claims

1. An artificial stent consists of a stent body and a coating thereon, wherein the coating comprises a drug-loaded layer containing silk fibroin and a drug, wherein the drug-loaded layer is of a microporous structure substantially formed with silk fibroin, for loading the drug.

2. The artificial stent according to claim 1, wherein the coating further comprises a dense base layer substantially formed with silk fibroin, between the stent body and the drug-loaded layer.

3. The artificial stent according to any one of claims 1-2, wherein the drug includes a water-soluble or lipid-soluble chemical drug.

4. The artificial stent according to claim 3, wherein the drug is a proteinous drug and/or a nucleic acid drug.

5. A method for the manufacture of the artificial stent according to claim 1, comprising the steps of
1) dissolving silk fibroin in water or an organic solvent mixing evenly, obtaining a silk fibroin solution of 1%wt-20%wt, and adding an aqueous gelatin solution, mixing evenly, collecting the supernatant after centrifugation;
2) uniformly coating the surface of the stent body with the supernatant in step 1), denaturing the stent by heat or chemical reagents, washing the stent with purified water, placing the stent into purified water after drying the same, boiling the stent until gelatin in the coating dissolves out completely, washing the stent with purified water, thereby obtaining a microporous structure drying of the coat;
3) placing the stent obtained in step 2) into the solution of a drug, so as to load the drug into the micropores in the coating; removing the stent and allowing it to dry.

6. The method according to claim 5, wherein the concentration of the gelatin solution in step 1) is 5-20% wt, and the volumetric ratio of the gelatin solution to the solution of silk fibrioin is no more than 1:1.

7. The method according to claim 5, wherein a further step of γ-ray irradiation, vacuum drying-heat treatment, and treatment with a monohydroxy alcohols or a polyhydroxy alcohol or combinations thereof, is further comprised after step 2).

8. A method for the manufacture of the artificial stent according to claim 1, comprising the steps of
1) dissolving silk fibroin in water or an organic solvent and mixing evenly, obtaining a silk fibroin solution of 1%wt-20%wt;
2) uniformly coating the surface of a stent body with the silk fibroin solution obtained in step 1), denaturing the stent by heat or chemical reagents, soaking the stent with purified water, freezing and warming-drying, so as to allow the formation of a microporous structure in the coating;
3) placing the stent obtained in step 2) into the solution of a drug, so as to load the drug into the micropores in the coating; removing the stent and allowing it to dry.

9. The method according to claim 8, wherein the freezing and warming-drying in step 2) is as follows: firstly, decreasing the temperature to -40°C ∼ -80°C with a programmed scheme, with a rate of -0.5°C/min ∼ -5°C/min, thereby allowing the crystallization of water in the coating; then increasing the temperature to room temperature with a programmed scheme with a rate of 0.1°C/min ∼ 2°C/min, under high vacuum, thereby allowing the complete drying of the coating.

10. The method according to claim 8, wherein a further step of γ-ray irradiation, vacuum drying-heat treatment, and treatment with a monohydroxy alcohols or a polyhydroxy alcohol or combinations thereof, is further comprised after step 2).

## Patentansprüche

1. Künstlicher Stent, bestehend aus einem Stentkörper und einer Beschichtung darauf, wobei die Beschichtung eine arzneimittelbeladene Schicht umfasst, die Seidenfibroin und ein Arzneimittel enthält, wobei die arzneimittelbeladene Schicht eine im Wesentlichen mit Seidenfibroin gebildete mikroporöse Struktur aufweist, um mit dem Arzneimittel beladen zu werden.

2. Künstlicher Stent nach Anspruch 1, wobei die Beschichtung ferner zwischen dem Stentkörper und der arzneimittelbeladenen Schicht eine dichte Basisschicht umfasst, die im Wesentlichen mit Seidenfibroin gebildet ist.

3. Künstlicher Stent nach einem der Ansprüche 1 bis 2, wobei das Arzneimittel ein wasserlösliches oder lipidlösliches chemisches Arzneimittel einschließt.

4. Künstlicher Stent nach Anspruch 3, wobei das Arzneimittel ein proteinartiges Arzneimittel und/oder ein Nukleinsäurearzneimittel ist.

5. Verfahren zur Herstellung des künstlichen Stents nach Anspruch 1, umfassend die folgenden Schritte:
1) Lösen von Seidenfibroin in Wasser oder einem organischen Lösungsmittel unter gleichförmigem Mischen, Erhalt einer 1 Gew.% bis 20 Gew.% Seidenfibroinlösung, und Hinzufügung einer wässrigen Gelatinelösung unter gleichförmigem Mischen, Auffangen des Überstands nach dem Zentrifugieren;
2) gleichförmiges Beschichten der Oberfläche des Stentkörpers mit dem Überstand von Schritt 1), Denaturieren des Stents mit Wärme oder chemischen Reagenzien, Waschen des Stents mit gereinigtem Wasser, Platzieren des Stents in gereinigtem Wasser nach Trocknen desselben, Kochen des Stents, bis die Gelatine in der Beschichtung vollständig herausgelöst ist, Waschen des Stents mit gereinigtem Wasser, wodurch eine mikroporöse Struktur erhalten wird, Trocknen der Beschichtung,
3) Platzieren des in Schritt 2) erhaltenen Stents in der Lösung eines Arzneimittels, um so die Mikroporen in der Beschichtung mit dem Arzneimittel zu beladen; Entfernen des Stents und Trocknenlassen des Stents.

6. Verfahren nach Anspruch 5, wobei die Konzentration der Gelatinelösung in Schritt 1) 5 bis 20 Gew.% beträgt und das volumetrische Verhältnis der Gelatinelösung zu der Lösung des Seidenfibroins nicht mehr als 1:1 beträgt.

7. Verfahren nach Anspruch 5, wobei nach Schritt 2) ein weiterer Schritt der Bestrahlung mit γ-Strahlen, Vakuumtrocknung-Wärmebehandlung und Behandlung mit einem ein- oder mehrwertigen Alkohol oder Kombinationen davon durchgeführt wird.

8. Verfahren zur Herstellung des künstlichen Stents nach Anspruch 1, umfassend die folgenden Schritte:
1) Lösen von Seidenfibroin in Wasser oder einem organischen Lösungsmittel unter gleichförmigem Mischen, Erhalt einer 1 Gew.% bis 20 Gew.% Seidenfibroinlösung;
2) gleichförmiges Beschichten der Oberfläche eines Stentkörpers mit der in Schritt 1) erhaltenen Seidenfibroinlösung, Denaturieren des Stents durch Wärme oder chemische Reagenzien, Einweichen des Stents in gereinigtem Wasser, Gefrieren und Wärmetrocknen, um so die Bildung einer mikroporösen Struktur in der Beschichtung zu ermöglichen;
3) Platzieren des in Schritt 2) erhaltenen Stents in der Lösung eines Arzneimittels, um so die Mikroporen in der Beschichtung mit dem Arzneimittel zu beladen; Entfernen des Stents und Trocknenlassen des Stents.

9. Verfahren nach Anspruch 8, wobei das Gefrieren und Wärmetrocknen in Schritt 2 folgendermaßen erfolgt: zuerst Verringern der Temperatur auf -40 °C bis -80 °C gemäß einem programmierten Schema mit einer Rate von -0,5 °C/Min bis -5 °C/Min, wodurch die Kristallisation von Wasser in der Beschichtung ermöglicht wird; danach Erhöhen der Temperatur auf Raumtemperatur gemäß einem programmierten Schema mit einer Rate von 0,1 °C/Min bis 2 °C/Min unter Hochvakuum, wodurch ein vollständiges Trocknen der Beschichtung möglich wird.

10. Verfahren nach Anspruch 8, wobei nach Schritt 2) ein weiterer Schritt der Bestrahlung mit γ-Strahlen, Vakuumtrocknung-Wärmebehandlung und Behandlung mit einem ein- oder mehrwertigen Alkohol oder Kombinationen davon durchgeführt wird.

## Revendications

1. Stent artificiel consistant en un corps de stent et d'un revêtement sur ce dernier, dans lequel le revêtement comprend une couche chargée de médicament et contenant une fibroïne de soie, et un médicament, dans lequel la couche chargée de médicament est une structure microporeuse substantiellement formée avec de la fibroïne de soie, pour charger le médicament.

2. Stent artificiel selon la revendication 1, dans lequel le revêtement comprend en outre une couche de base dense substantiellement formée avec de la fibroïne de soie, entre le corps de stent et la couche chargée de médicament.

3. Stent artificiel selon l'une quelconque des revendications 1 à 2, dans lequel le médicament comprend un médicament chimique soluble dans l'eau ou soluble dans les lipides.

4. Stent artificiel selon la revendication 3, dans lequel le médicament est un médicament protéique et/ou un médicament à base d'acide nucléique.

5. Procédé pour fabriquer le stent artificiel selon la revendication 1, comprenant les étapes :
1) dissoudre la fibroïne de soie dans l'eau ou dans un solvant organique, mélanger régulièrement, obtenir une solution de fibroïne de soie de 1% à 20% en poids et ajouter une solution de gélatine aqueuse, mélanger régulièrement, collecter le surnageant après centrifugation ;
2) recouvrir uniformément la surface du corps de stent avec le surnageant de l'étape 1), dénaturer le stent par chauffage ou avec des réactifs chimiques, laver le stent avec de l'eau purifiée, placer le stent dans l'eau purifiée après l'avoir séché, faire bouillir le stent jusqu'à ce que la gélatine dans le revêtement se dissolve complètement, laver le stent avec l'eau purifiée, obtenir ainsi une structure microporeuse, faire sécher le revêtement ;
3) placer le stent obtenu à l'étape 2) dans la solution de médicament, afin de charger le médicament dans les micropores du revêtement : retirer le stent et le laisser sécher.

6. Procédé selon la revendication 5, dans lequel la concentration de solution de gélatine de l'étape 1) est de 5 à 20% en poids, et le rapport volumétrique de la solution de gélatine sur la solution de fibroïne de soie n'est pas supérieur à 1 : 1.

7. Procédé selon la revendication 5, dans lequel une autre étape de rayonnement aux rayons γ, de traitement de séchage thermique sous vide et de traitement avec des alcools monohydroxyliques ou un alcool polyhydroxylique ou leurs combinaisons, est en outre comprise après l'étape 2).

8. Procédé pour fabriquer le stent artificiel selon la revendication 1, comprenant les étapes :
1) dissoudre la fibroïne de soie dans l'eau ou dans un solvant organique et mélanger régulièrement, obtenir une solution de fibroïne de soie de 1% à 20% en poids ;
2) recouvrir uniformément la surface d'un corps de stent avec la solution de fibroïne de soie obtenue à l'étape 1), dénaturer le stent par chauffage ou avec des réactifs chimiques, laisser tremper le stent dans l'eau purifiée, congeler et sécher à chaud, afin de permettre la formation d'une structure microporeuse dans le revêtement ;
3) placer le stent obtenu à l'étape 2) dans la solution de médicament, afin de charger le médicament dans les micropores du revêtement ; retirer le stent et le laisser sécher.

9. Procédé selon la revendication 8, dans lequel l'étape de congélation et de séchage à chaud à l'étape 2) consiste à : premièrement, abaisser la température de - 40°C ∼ 80°C avec un procédé programmé, avec une vitesse de - 0,5°C/min ∼ - 5°C/min, permettant ainsi la cristallisation de l'eau dans le revêtement ; faire monter ensuite la température à la température ambiante avec un procédé programmé avec une vitesse de 0,1°C/min ∼ 2°C/min, sous vide poussé, permettant ainsi le séchage complet du revêtement.

10. Procédé selon la revendication 8, dans lequel une étape supplémentaire de rayonnement aux rayons γ, de traitement de séchage thermique sous vide et de traitement avec des alcools monohydroxyliques ou un alcool polyhydroxylique ou leurs combinaisons, est en outre comprise après l'étape 2).
